# FASCICULE DE BREVET EUROPEEN

(11) **EP 1 056 872 B1**
(45) Date de publication et mention de la délivrance du brevet: **24.08.2005**
(21) Numéro de dépôt: 99961178.3
(22) Date de dépôt: 28.12.1999
(51) Int. Cl.: C12N 15/74, C12N 15/87, C12N 1/21

(54) **SEQUENCES D'ADN CONTENANT UN MECANISME DE TRANSFERT PAR CONJUGAISON**
DNA SEQUENZEN FÜR KONJUGATIONSTRANSFERMECHANISMUS
DNA SEQUENCES CONTAINING A TRANSFER MECHANISM BY CONJUGATION

(30) Priorité: 29.12.1998 FR 9816529
(43) Date de publication de la demande: 06.12.2000
(73) Titulaire: BIOGHURT BIOGARDE GMBH & CO. KG, 85354 Freising (DE)
(72) Inventeur: PREVOTS, Fabien, F-31450 Donneville (FR); DALOYAU, Marlène, F-31450 Montgiscard (FR)
(74) Mandataire: Nevant, Marc
(86) Numéro de dépôt international: PCT/FR1999/003297
(87) Numéro de publication internationale: WO 2000/039312

(56) Documents cités:
- EP-A- 0 316 677
- MILLS D.A. ET AL.: "Genetic analysis of regions of the Lactococcus lactis subsp. lactis plasmid pRS01 involved in conjugative transfer." APPL. ENVIRON. MICROBIOL., vol. 60, no. 12, décembre 1994 (1994-12), pages 4413-4420, XP002114300 cité dans la demande
- O'CONNOR L. ET AL.: "AbiG, a genotipically novel abortive infection mechanism encoded by plamid pCI750 of Lactococcus lactis subsp. cremoris UC653" APPL. ENVIRON. MICROBIOL., vol. 62, no. 9, septembre 1996 (1996-09), pages 3075-3082, XP002114301
- HILL C ET AL: "THE CONJUGATIVE PLASMID PTR2030 ENCODES TWO BACTERIOPHAGE DEFENSE MECHANISMS IN LACTOCOCCI, RESTRICTION MODIFICATION (R+/M+) AND ABORTIVE INFECTION (HSP+)" APPLIED AND ENVIRONMENTAL MICROBIOLOGY, vol. 55, no. 9, 1 septembre 1989 (1989-09-01), pages 2416-2419, XP000749837 ISSN: 0099-2240

## Description

La présente invention a pour objet des nouvelles séquences d'ADN susceptibles d'être transférées par conjugaison, les plasmides contenant ces séquences, les bactéries contenant ces séquences d'ADN ou ces plasmides, ainsi que l'utilisation de ces bactéries.

Les bactéries lactiques sont impliquées dans l'élaboration et la conservation d'un grand nombre de produits alimentaires, tels que les fromages, le beurre, les yaourts, le saucisson ou la choucroute. Parmi ceux-ci les produits laitiers occupent une place particulièrement importante. La transformation du lait par les bactéries lactiques est faite dans des cuves de plus en plus grandes. La compréhension des mécanismes de transfert de matériel génétique est essentielle afin d'améliorer les souches de bactéries lactiques utilisées dans ces fermentations. Les différents mécanismes de transfert de matériel génétique sont : la transformation, la transduction, la fusion de protoplastes et la conjugaison.

La transformation consiste à faire pénétrer du matériel génétique dans une bactérie, par compétence naturelle, par protoplastisation ou électroperméation des cellules.

La transduction consiste à faire pénétrer du matériel génétique dans une bactérie au moyen d'un bactériophage-vecteur.

La fusion de protoplastes consiste à protoplastiser deux types cellulaires, de telle sorte qu'après contact, du matériel génétique d'une souche passe dans l'autre souche.

La conjugaison consiste à mettre en contact deux types cellulaires, de telle sorte que du matériel génétique de l'une des souches passe dans l'autre souche grâce à des gènes naturels de conjugaison.

On peut distinguer deux cas : soit le plasmide possède tous les gènes impliqués dans la conjugaison et il passe lui-même dans la souche réceptrice, soit le plasmide ne possède que les éléments génétiques suffisants pour son transfert, les autres éléments étant présent par exemple sur un autre plasmide (Mobilization of the relaxable *Staphylococcus aureus* plasmid pC221 by the conjugative plasmid pG01 involves three pC221 loci, S.J. Projan et G.L. Archer, J. Bacteriol. 1989 ; 171: 1841-1845). Cette dernière situation présente un avantage : si le plasmide est transféré dans une souche réceptrice sans le plasmide conjugatif, il ne pourra plus être transféré de nouveau, ce qui évitera sa dissémination. Cette technique de transfert de plasmide par conjugaison présente également l'avantage de faire passer du matériel génétique dans des souches dans lesquelles cela est difficile par les techniques de transfert décrites précédemment.

Quelques études ont déjà permis de mettre en évidence des systèmes de conjugaison chez les bactéries lactiques:
- Genetic analysis of régions of the *Lactococcus lactis* subsp. *lactis* plasmid pRS01 involved in conjugative transfer, D.A Mills, C.K. Choi, G.M. Dunny et L.L. McKay, Applied and Environ. Microbiol. 1994 ; 60(12) : 4413-4420, ce document divulgue la localisation de quatre régions non adjacentes du plasmide pRS01 de *Lactococcus lactis* ssp. *lactis* ML3, impliquées dans le transfert conjugatif. Les séquences nudéotidiques de ces régions n'ont pas été déterminées et n'ont pas fait l'objet d'un processus d'isolement.
- Splicing of a group II intron involved in the conjugative transfer of pRS01 in lactococci, D.A. Mills, L.L. McKay et G.M. Dunny., J. Bacteriol. 1996 ; 178 (12): 3531-3538.
- Sequence and analysis of the 60 kb conjugative, bacteriocin-producing plasmid pMRC01 from *Lactococcus lactis* DPC3147, B.A. Dougherty, C. Hill, J.F. Weidman, D.R. Richardson, J. C. Venter and R. P. Ross, Molecular Microbiology 1998, vol. 29(4): 1029-38. Ce document décrit la séquence complète du plasmide conjugatif pMRC01 de *L. lactis* ssp. *lactis* DPC 3147. Parmi les trois régions fonctionnelles identifiées dans ce plasmide, la région impliquée dans le transfert conjugatif comprend un opéron, composé de 16 gènes, qui est présumé respénsable des fonctions conjugatives du plasmide pMRC01.

Dans la présente demande, une séquence d'ADN comprenant au moins un mécanisme de transfert par conjugaison est décrite; cette séquence d'ADN comprend une partie fonctionnelle de 5333 pb présente dans la souche *Lactococcus lactis* FL877 déposée le 30 septembre 1998 à la CNCM (Collection Nationale de Cultures de Microorganismes) sous le n° I-2082.

Cette séquence d'ADN de 5333 pb (SEQ ID N° : 1) a été isolée à partir des plasmides contenus dans la souche FL877.

Plus précisément, le fragment de 5333 paires de bases (pb) a été isolé par une digestion totale à l'aide de l'enzyme de restriction *Eco*RI des plasmides contenus dans la souche *Lactococcus lactis* FL877. Ce fragment est porteur d'un ou plusieurs mécanismes de transfert par conjugaison et est susceptible d'être transféré dans une autre souche, notamment une autre souche de *L*. *lactis*, à partir par exemple de la souche MG1363 (GASSON M.J., J. Bacteriol. 1983 ; 154: 1-9). Ce fragment est également porteur d'un système de réplication fonctionnelle chez *L. lactis*.

Puis, à partir de la séquence nucléotidique SEQ ID N° : 1, la Demanderesse a isolé une séquence d'ADN de 2 590 pb, qui confère à elle seule la propriété à un plasmide d'être transféré dans une autre souche par conjugaison à partir notamment de la souche MG1363.

Cette séquence de 2590 pb (SEQ ID N° : 2) peut être obtenue par la méthode de la PCR (polymerase chain reaction) à l'aide d'oligonucléotides appropriés.

Ainsi, selon un premier aspect, la présente invention a pour objet une séquence d'acide nucléique susceptible d'être transférée par conjugaison, qui comprend la séquence SEQ ID N° : 2, ou son brin complémentaire.

De préférence l'invention a pour objet une séquence d'acide nucléique susceptible d'être transférée par conjugaison, ladite séquence étant choisie parmi :
a) la séquence nucléotidique de 5333 pb (SEQ ID N° : 1) ou son brin complémentaire ;
b) toute séquence hybridant avec la séquence a) dans des conditions strictes.

Plus particulièrement, l'invention concerne une séquence d'acide nucléique qui est choisie parmi :
a) la séquence nucléotidique de 2590 pb (SEQ ID N° : 2) ou son brin complémentaire ;
b) toute séquence hybridant avec la séquence a) dans des conditions strictes.

Selon la présente invention, on entend par "hybridant dans des conditions strictes" une hybridation dans les conditions de stringence suivantes : 42°C, dans un tampon phosphate de sodium 20 mM (pH 6,5) contenant 50 % de formamide, 5 x SSC, 1 x Denhardt's, 0,1 % de SDS et 100 µg/ml d'ARN, puis lavage à 60°C dans un tampon 0,1 x SSC, 0,1 % SDS.

L'invention a également pour objet les plasmides transformés avec l'une des séquences d'ADN selon l'invention. Ces plasmides peuvent être par exempte les plasmides pLDP1 (PREVOTS F. et al, FEMS Microbiol. Lett. 1996 ; 142: 295-299) et pLAB510, dérivé de pPF107-3 (PREVOTS F. et al. FEMS Microbiol. Lett. 1998 ; 159: 331-336) dans lesquels on a cloné, selon les techniques habituelles bien connues de l'homme de l'art, l'une des séquences d'ADN selon l'invention.

L'invention a également trait aux bactéries, notamment aux bactéries lactiques, de préférence appartenant à l'espèce *Lactococcus lactis*, qui contiennent au moins une des séquences d'ADN ou un plasmide tels que définis ci-dessus.

Ces bactéries peuvent être utilisées pour transmettre par conjugaison du matériel génétique, ayant notamment un intérêt industriel, à une souche d'intérêt industriel. Le mécanisme de transfert par conjugaison peut être porté par un plasmide ou par une autre partie du génome de la bactérie.

En particulier, ces bactéries peuvent être utilisées pour transférer par conjugaison des propriétés telles que la résistance aux phages, l'aptitude à fermenter le lactose, la protéolyse, la peptidolyse, la production de bactériocines, et des gènes codant pour des protéines d'intérêt pharmaceutiques, à des souches d'intérêt industriel, en particulier dans l'industrie laitière, mais également dans l'industrie pharmaceutique.

Les souches d'intérêt industriel qui peuvent avantageusement recevoir du matériel génétique à l'aide des séquences d'ADN selon l'invention ou d'un plasmide les contenant sont choisies parmi les souches *L. lactis* ssp *lactis* et *L. lactis* ssp *cremoris*.

L'invention sera mieux comprise à l'aide des exemples ci-après, qui comprennent des résultats expérimentaux et une discussion de ceux-ci. Certains de ces exemples concernent des expériences effectuées dans le but de réaliser l'invention, d'autres concernent des exemples de réalisation de l'invention donnés bien sûr à titre purement illustratif.

Une grande partie de l'ensemble des techniques décrites dans ces exemples, bien connu de l'homme de l'art, est exposée en détails dans l'ouvrage de Sambrook, Frittsch et Maniatis : "Molecular Cloning; a laboratory manual" publié en 1989 par les éditions Cold Srping Harbor Press à New York (2e édition).

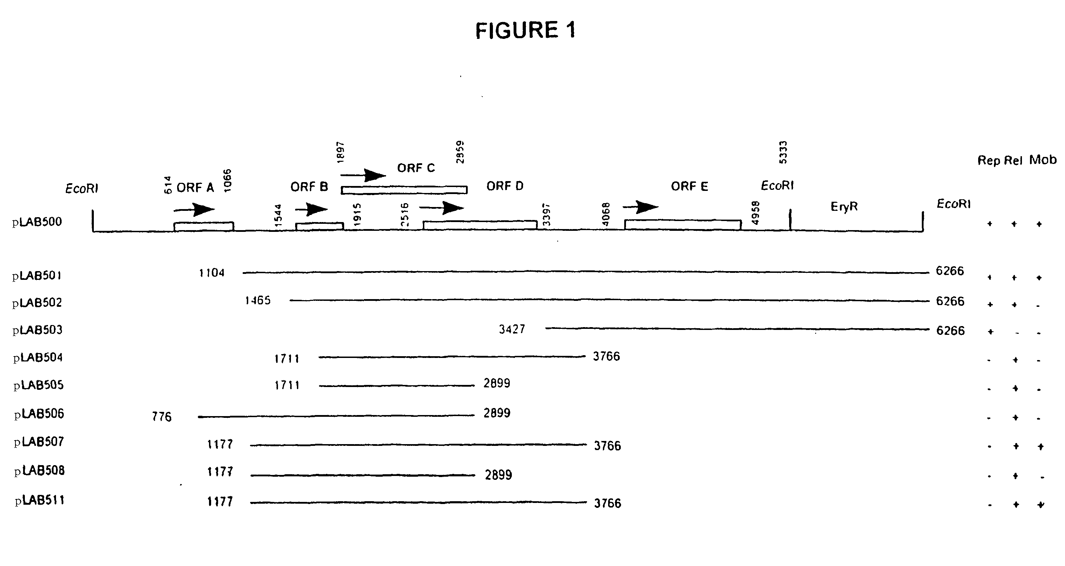

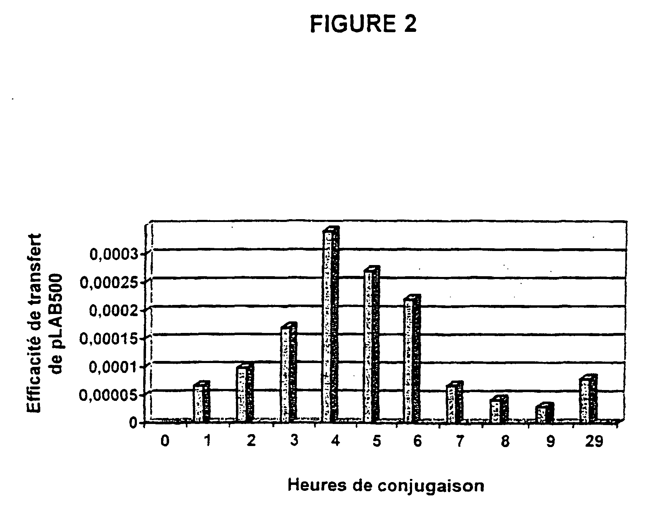

La description ci-après sera mieux comprise à l'aide des figures 1 et 2, ci-après qui représentent respectivement :
Figure 1 : Sous-clonage de fragments internes du plasmide pLAB500.
   Dans cette figure, on utilise les abréviations suivantes :
   Rep+ ou - : fragment réplicatif (+) ou non (-) dans MG1363.
   Rel+ ou - : relaxation (+) ou non (-) dans MG1363.
   Mob+ ou - : haute (+) ou faible (-) efficacité de transfert du plasmide de MG1363 à LM2301.

   Par ailleurs, les chiffres au-dessus des séquences représentent la position en base sur le plasmide pLAB500.
Figure 2 : Cinétique de transfert du plasmide pLAB500.

### Exemple 1 : séquence du fragment de 5333 pb.

La technique de la PCR (polymerase chain reaction), décrite par exemple dans l'ouvrage de Maniatis, déjà cité, permet d'amplifier un fragment d'ADN compris entre 2 oligonucléotides convenablement choisis. Cet ADN amplifié peut être aisément ligaturé sur lui-même si des sites de restriction sont apportés par les oligonucléotides. En effet, les séquences de ces oligonucléotides peuvent comporter, à leur extrémité 5', une partie hétérologue de l'ADN à amplifier, constituée par exemple de 10 à 12 paires de bases, dont six constituent un site de restriction.

Les oligonucléotides suivants ont été utilisés pour la construction d'un gène de résistance à l'érythromycine bordé de part et d'autre d'un site de restriction *Eco*RI :
oligonucléotide 1 :
oligonucléotide 2 :
oligonucléotide 3 :
oligonucléotide 4 :
oligonucléotide 5 :
oligonucléotide 6 :
oligonucléotide 7 :
oligonucléotide 8 :

Les oligonucléotides 5 et 1 ont permis d'amplifier par PCR, à partir du plasmide pRC1 (LE BOURGEOIS P. et al., Gene 1992 ; 111: 109-114) un fragment de 1,1 kb contenant l'origine de réplication.

Les oligonucléotides 2 et 6 ont permis d'amplifier par PCR, à partir du plasmide pRC1, un fragment de 1 kb contenant le gène de résistance à l'érythromycine.

Ces deux fragments ont été digérés par *Bcl*I et *Mlu*I, ligaturés, puis ont servis à transformer *E. coli* TG1 (GIBSON T.J., Studies on the Epstein-Barr genome, Ph.D thesis, Cambridge University, Cambridge, UK, 1984) pour donner le plasmide pRC1int.

Les oligonucléotides 3 et 4 ont permis d'amplifier par PCR, à partir du plasmide pRC1, un fragment de 0,3 kb contenant plusieurs sites uniques de restriction. Ce fragment a été digéré par *Bcl*I, puis ligaturé au plasmide pRC1int lui-même digéré par *Bcl*I. Ce nouveau plasmide obtenu, contenant le gène de résistance à l'érythromycine, une origine de réplication fonctionnelle chez *E. coli* mais pas chez *L. lactis* et plusieurs sites uniques de restriction a été nommé pRC1N.

Les oligonucléotides 7 et 8 ont été utilisés pour amplifier par PCR, à partir du plasmide pRC1N, un fragment de 939 pb contenant le gène de résistance à l'érythromycine. Ce fragment a ensuite été digéré par *Eco*RI puis ligaturé à l'ensemble des plasmides de la souche *L. lactis* FL877 préalablement extraits et digérés par l'enzyme de restriction *Eco*RI. La souche MG1363 a été transformé par ce mélange de ligation. L'ensemble des clones résistants à l'érythromycine ainsi obtenus a été utilisé comme souche donneuse dans une expérience de conjugaison (PREVOTS F. et al., FEMS Microbiol. Lett. 1994 ; 117: 7-14) avec la souche réceptrice LM2301 (WALSH P.M. et al., J. Bacteriol. 1981 ; 146: 937-944), résistante à la streptomycine et sensible à l'érythromycine. Les transconjugants sont sélectionnés pour leur résistance à ces deux antibiotiques. Les plasmides ayant été transférés par conjugaison dans cette souche LM2301 ont été extraits et ont servi à retransformer la souche MG1363. Le plasmide pLAB500 de 6,3 kb a ainsi été isolé, capable de se répliquer aussi bien dans les souches LM2301 et MG1363, et capable d'être transféré de la souche MG1363 à la souche LM2301 avec une haute efficacité.

Après digestion à l'enzyme de restriction *Eco*RI, ce plasmide donne 2 fragments de 1 kb et 5,3 kb. Le premier fragment contient le gène de résistance à l'érythromycine. Le dernier fragment de 5333 pb a été entièrement séquencé par la méthode de Sanger et al. (PNAS - USA 1977; 14: 5463).

L'analyse de la séquence a montré que le fragment de 5333 pb possède 5 phases de lecture ouverte complètes de plus de 150 pb (Fig. 1).

### Exemple 2 : diminution de la taille du plasmide pLAB500.

La technique PCR a été appliquée en vue de déterminer quels sont les cadres ouverts de lecture impliqués dans la conjugaison, et ceux impliqués dans la réplication du plasmide.

Pour cela, 9 oligonucléotides comprenant chacun un site de restriction *Eco*RI ou *Hind*III ont été synthétisés. La séquence de ces oligonucléotides est la suivante :
oligonucléotide 9 :
oligonucléotide 10 :
oligonucléotide 11 :
oligonucléotide 12 :
oligonucléotide 13 :
oligonucléotide 14:
oligonucléotide 15 :
oligonucléotide 16 :
oligonucléotide 17 :

Les oligonucléotides 14 et 15 ont permis d'amplifier un fragment d'ADN de 2846 pb comportant le gène de résistance à l'érythromycine et l'ORF E sous la forme d'un fragment *Hind*III-*Hind*III, grâce aux sites de restriction apportés par les oligonucléotides, permettant une ligaturation de ce fragment sur lui-même.

De la même manière, les oligonucléotides 14 et 16 ont permis d'amplifier un fragment d'ADN de 4808 pb comportant le gène de l'érythromycine et les ORF B, C, D et E.

Enfin, les oligonucléotides 14 et 17 ont permis d'amplifier d'amplifier un fragment d'ADN de 5169 pb comportant le gène de l'érythromycine et les ORF B, C, D et E.

Ces fragments d'ADN ont été amplifiés par PCR à partir d'une préparation de plasmide pLAB500 avec l'enzyme ELONGASE® (BRL).

Les produits de PCR ont été purifiés par une extraction au phénol-chloroforme, précipités à l'éthanol, digérés par l'enzyme de restriction *Hind*III, ligaturés sur eux-mêmes. Le clonage de ces fragments sur eux-mêmes a conduit aux plasmides pLAB501, pLAB502 et pLAB503. Ces plasmides ont été construits dans la souche MG1363. Leur aptitude à se répliquer dans cette souche indique que l'ORF E code pour une protéine impliquée dans la réplication du plasmide. D'ailleurs, cette protéine montre 21.7 % d'homologie sur 230 acides aminés avec la protéine RepE impliquée dans la réplication du facteur F de *E. coli*.

Ces plasmides ont ensuite été testé pour leur aptitude à être transférés par conjugaison dans la souche LM2301. Les plasmides pLAB502 et pLAB503 ont une efficacité de transfert comparable à pLDP1, le témoin négatif. On peut donc dire que ces plasmides ne possèdent pas la totalité de l'ADN nécessaire à leur transfert par conjugaison. Par contre, pLAB501 a une efficacité de transfert par conjugaison comparable à pLAB500 : l'ensemble de l'ADN nécessaire au transfert par conjugaison de pLAB500 doit donc être présent dans pLAB501.

### Exemple 3 : sous-clonage de fragments de pLAB500 dans le plasmide pLDP1.

Les oligonucléotides 9 et 12 ont permis d'amplifier par PCR un fragment comportant les ORF C et D sous la forme d'un fragment *Eco*RI-*Eco*RI de 2068 pb.

Les oligonucléotides 9 et 13 ont permis d'amplifier par PCR un fragment comportant l'ORF C sous la forme d'un fragment *Eco*RI-*Eco*RI de 1201 pb.

Les oligonucléotides 11 et 13 ont permis d'amplifier par PCR un fragment comportant les ORF B et C sous la forme d'un fragment *Eco*RI-*Eco*RI de 2136 pb.

Les oligonucléotides 10 et 12 ont permis d'amplifier par PCR un fragment comportant les ORF B, C et D sous la forme d'un fragment *Eco*RI-*Eco*RI de 2602 pb.

Les oligonucléotides 10 et 13 ont permis d'amplifier par PCR un fragment comportant les ORF B et C sous la forme d'un fragment *Eco*RI-*Eco*RI de 1735 pb.

Chacun de ces fragments, après digestion par l'enzyme *Eco*RI, a été cloné dans pLDP1, donnant respectivement les plasmides pLAB504, pLAB505, pLAB506, pLAB507 et pLAB508. Parmi ces plasmides, seul pLAB507 a été transféré avec une efficacité significative, meilleure que les autres plasmides, mais cependant moins bonne que pLAB500. On peut donc dire que les ORF B, C et D sont nécessaires au transfert par conjugaison.

### Exemple 4 : sous-clonage d'un fragment de pLAB500 dans pLAB510.

Les oligonucléotides suivants ont été utilisés dans ce sous-clonage :
oligonucléotide 18 :
oligonucléotide 19 :
oligonucléotide 20 :
oligonucléotide 21 :

Le plasmide pLDP1 est transféré par conjugaison avec une efficacité faible mais non nulle de MG1363 à LM2301. Afin d'éliminer l'apport de ce plasmide dans le transfert, le fragment de PCR obtenu avec les oligonucléotides 10 et 12 a été cloné dans un autre plasmide.

Le gène de résistance à l'érythromycine a d'abord été amplifié par PCR à partir du plasmide pRC1N et des oligonucléotides 18 et 19, puis digéré par les enzymes de restriction *Bam*HI et *Eco*RI. Un fragment réplicatif du plasmide pPF107-3 (numéro d'accession GenBank : Y12675) a été amplifié grâce aux oligonucléotides 20 et 21 à partir de l'ADN total de la souche I-942 (déposée à la C.N.C.M. le 12 avril 1990), puis digéré par les enzymes de restriction *Bam*HI et *Eco*RI. Ces deux fragments de PCR ont été ligaturés et ont servi à transformer MG1363. Le nouveau plasmide obtenu, appelé pLAB510, a été digéré par *Eco*RI, puis ligaturé avec le fragment obtenu avec les oligonucléotides 10 et 12, obtenu par amplification PCR à partir du plasmide pLAB500, et digéré par *Eco*RI. Cette ligature a servis à transformer MG1363. Ce nouveau plasmide, pLAB511, a été testé pour son aptitude à être transféré de MG1363 à LM2301 (tableau 1). On peut constater que ce nouveau plasmide pLAB511 est bien transférable entre ces deux souches par conjugaison.

**Tableau 1 :**

| Efficacité de transfert par conjugaison | | | |
|---|---|---|---|
| **Plasmide** | **ORF** | **Efficacité** | **Efficacité relative** |
| pLAB500 | A,B,C,D,E | 8,3 x 10⁻⁴ | 100 |
| pLAB501 | B,C,D,E | 7,1 x 10⁻⁴ | 85 |
| pLAB502 | B,C,D,E | 1,1 x 10⁻⁷ | 0,01 |
| pLAB503 | E | < 5 x 10⁻⁹ | < 0,0006 |
| pLAB504 | C,D | 1 x 10⁻⁷ | 0,01 |
| pLAB505 | C,D | 5,7 x 10⁻⁸ | 0,007 |
| pLAB506 | B,C | 5,7 x 10⁻⁸ | 0,007 |
| pLAB507 | B,C,D | 4,5 x 10⁻⁵ | 5,4 |
| pLAB508 | B,C | 4,2 x 10⁻⁷ | 0,05 |
| pLDP1 | / | 9,2 x 10⁻⁸ | 0.01 |
| pLAB510 | / | < 5 x 10⁻⁹ | < 0,0006 |
| pLAB511 | B,C,D | 1,3 x 10⁻³ | 157 |
| Efficacité = rapport du nombre de transconjugants sur le nombre de réceptrices. | | | |
| Efficacité relative = rapport de l'efficacité de conjugaison pour un plasmide donné sur l'efficacité de conjugaison pour pLAB500 x 100. | | | |
| ORF = phases de lecture ouverte entières contenues dans le plasmide. | | | |

### Exemple 5 : mise en évidence de la relaxation conféré par différents plasmides.

La relaxation (formation de formes circulaires ouvertes de plasmides à partir de plasmides à forme superenroulée) est impliquée dans le transfert de certains plasmides (Plasmid-protein relaxation complexes in *Staphylococcus aureus*., R. Novick., J. Bacteriol. 1976 ; 127:1177-1187). Par les comparaisons avec les banques de données informatiques (GenBank) la protéine déduite de l'ORF C montre 35,2 % d'homologie avec Rlx, une relaxase de *Staphylococcus aureus*. On peut donc supposer que cette protéine déduite de l'ORF C est également une relaxase. Les plasmides pLAB500 à pLAB508, pLAB510, pLAB511 et pLDP1 ont donc été extraits et déposés sur gel d'agarose. Après migration et parmi tous ces plasmides extraits de MG1363, seuls pLAB503, pLAB510 et pLDP1 ne présentent pas de forme circulaire ouverte : l'ORF C est donc bien impliquée dans la relaxation de plasmides.

### Exemple 6 : cinétique de transfert de pLAB500.

Des conjugaisons entre MG1363 contenant pLAB500 et LM2301 ont été effectuées à différents temps afin d'évaluer à partir de quand avait lieu le transfert. On constate (voir tableau 2 ci-après et figure 2) qu'après 1 heure, la conjugaison a déjà débuté et qu'un pic d'efficacité de transfert était atteint vers 4 heures. Des conjugaisons de 10 minutes en 10 minutes entre 0 et une heure ont également montré qu'aucune conjugaison n'avait lieu avant une heure.

**Tableau 2 :**

| Cinétique de transfert de pLAB500. | |
|---|---|
| **Temps de conjugaison (heures)** | **Efficacité de conjugaison** |
| 0 | < 1,1 x 10⁻⁸ |
| 1 | 6,6 x 10⁻⁵ |
| 2 | 9,8 x 10⁻⁵ |
| 3 | 1,7 x 10⁻⁴ |
| 4 | 3,4 x 10⁻⁴ |
| 5 | 2,7 x 10⁻⁴ |
| 6 | 2,2 x 10⁻⁴ |
| 7 | 6,7 x 10⁻⁵ |
| 8 | 4,2 x 10⁻⁵ |
| 9 | 3,0 x 10⁻⁵ |
| 29 | 7,9 x 10⁻⁵ |

Efficacité de conjugaison = rapport du nombre de transconjugants sur le nombre de réceptrices.

## Revendications

1. Séquence d'acide nucléique susceptible d'être transférée par conjugaison, qui comprend la séquence SEQ ID N° : 2 ou son brin complémentaire.

2. Séquence d'acide nucléique selon la revendication 1, qui est choisie parmi :
a) la séquence nucléotidique de 5333 pb (SEQ ID N° : 1) ou son brin complémentaire ;
b) toute séquence hybridant avec la séquence a) dans les conditions de stringence suivantes : 42°C, dans un tampon phosphate de sodium 20 mM (pH 6,5) contenant 50% de formamide, 5 x SSC, 1 x Denhardt's, 0,1 % de SDS et 100 µg/ml d'ARN, puis lavage à 60°C dans un tampon 0,1 x SSC, 0,1% SDS.

3. Séquence d'acide nucléique selon la revendication 1, qui est choisie parmi :
a) la séquence de 2590pb (SEQ ID N° : 2) ou son brin complémentaire ;
b) toute séquence hybridant avec la séquence a) dans les conditions de stringence suivantes : 42°C, dans un tampon phosphate de sodium 20 mM (pH 6,5) contenant 50% de formamide, 5 x SSC, 1 x Denhardt's, 0,1 % de SDS et 100 µg/ml d'ARN, puis lavage à 60°C dans un tampon 0,1 x SSC, 0,1% SDS.

4. Plasmide comprenant une séquence selon l'une des revendications 1 à 3.

5. Plasmide selon la revendication 4, qui est choisi parmi le plasmide pLDP1 ou le plasmide pLAB510.

6. Bactérie qui contient un plasmide selon la revendication 4 ou 5.

7. Bactérie selon la revendication 6, qui est une bactérie lactique.

8. Bactérie selon la revendication 7, qui appartient à l'espèce *Lactococcus lactis*.

9. Procédé pour transmettre du matériel génétique à une souche d'intérêt industriel choisie parmi les souches *L. lactis* ssp *lactis* ou *L. lactis* ssp *cremoris,* qui consiste à transférer par conjugaison le matériel génétique d'une bactérie selon la revendication 8 vers ladite souche d'intérêt industriel.

## Patentansprüche

1. Nucleinsäure-Sequenz, die durch Konjugation transferiert werden kann und die die Sequenz SEQ ID N°: 2 oder ihren komplementären Strang enthält.

2. Nucleinsäure-Sequenz nach Anspruch 1, die ausgewählt ist unter:
a) der Nucleotidsequenz von 5333 bp (SEQ ID N°: 1) oder ihrem komplementären Strang;
b) einer beliebigen Sequenz, die unter den folgenden Stringenzbedingungen mit der Sequenz a) hybridisiert: 42 °C, in Natriumphosphatpuffer 20 mM (pH 6,5) mit 50 % Formamid, 5 x SSC, 1 x Denhardt's, 0,1 % SDS und 100 µg/ml RNA, anschließend Waschen in einem Puffer 0,1 x SSC, 0,1 % SDS bei 60 °C.

3. Nucleinsäure-Sequenz nach Anspruch 1, die ausgewählt ist unter:
a) der Sequenz von 2590 bp (SEQ ID N°: 2) oder ihrem komplementären Strang;
b) einer beliebigen Sequenz, die unter den folgenden Stringenzbedingungen mit der Sequenz a) hybridisiert: 42 °C, in Natriumphosphatpuffer 20 mM (pH 6,5) mit 50 % Formamid, 5 x SSC, 1 x Denhardt's, 0,1 % SDS und 100 µg/ml RNA, anschließend Waschen in einem Puffer 0,1 x SSC, 0,1 % SDS bei 60 °C.

4. Plasmid, das eine Sequenz nach einem der Ansprüche 1 bis 3 enthält.

5. Plasmid nach Anspruch 4, das unter dem Plasmid pLDP1 oder dem Plasmid pLAB510 ausgewählt ist.

6. Bakterium, das ein Plasmid nach Anspruch 4 oder 5 enthält.

7. Bakterium nach Anspruch 6, bei dem es sich um ein Milchsäurebakterium handelt.

8. Bakterium nach Anspruch 7, das zur Spezies *Lactococcus lactis* gehört.

9. Verfahren zur Übertragung von genetischem Material auf einen industriell interessanten Stamm, der unter den Stämmen *L. lactis ssp lactis* oder *L. lactis ssp cremoris* ausgewählt ist, das darin besteht, das genetische Material eines Bakteriums nach Anspruch 8 auf den industriell interessanten Stamm durch Konjugation zu übertragen.

## Claims

1. Nucleic acid sequence capable of being transferred by conjugation, which comprises the sequence SEQ ID No:2, or its complementary strand.

2. Nucleic acid sequence according to claim 1, which is selected from:
a) the nucleotide sequence of 5333 bp (SEQ ID No:1) or its complementary strand;
b) any sequence hybridizing with the sequence a) under the following stringency conditions: 42°C in a 20 mM sodium phosphate buffer (pH 6.5) containing 50% of formamide, 5 x SSC, 1 x Denhardt's, 0.1% of SDS and 100 µg/ml of RNA, and then washing at 60°C in a buffer containing 0.1 x SSC and 0.1% of SDS.

3. Nucleic acid sequence according to claim 1, which is selected from:
a) the nucleotide sequence of 2590 bp (SEQ ID No:2) or its complementary strand;
b) any sequence hybridizing with the sequence a) under the following stringency conditions: 42°C in a 20 mM sodium phosphate buffer (pH 6.5) containing 50% of formamide, 5 x SSC, 1 x Denhardt's, 0.1% of SDS and 100 µg/ml of RNA, and then washing at 60°C in a buffer containing 0.1 x SSC and 0.1% of SDS.

4. Plasmid construct comprising a sequence according to any one of claims 1 to 3.

5. Plasmid according to claim 4, which is selected from plasmid pLDP1 or plasmid pLAB510.

6. Bacterium which contains a plasmid according to claim 4 or 5.

7. Bacterium according to claim 6, which is a lactic acid bacterium.

8. Bacterium according to claim 7, which belongs to the species *Lactococcus lactis*.

9. A method of transferring genetic material to a strain of industrial interest, selected from the strains *L. lactis* ssp *lactis* or *L. lactis* ssp *cremoris,* which consists in transferring the genetic material by conjugation from a bacterium according to claim 8 to said strain of industrial interest.


### LISTE DE SEQUENCES

<110> BIOGHURT BIOGARDE GmbH Co. KG
<120> Séquences d'ADN contenant un mécanisme de transfert par conjugaison, plasmides contenant une telle séquence, et bactéries contenant une telle séquence ou un tel plasmide
<130> h18957-38WO
<140>
   <141>
<150> FR98 16529
   <151> 1998-12-29
<160> 23
<170> PatentIn Ver. 2.1
<210> 1
   <211> 5333
   <212> ADN
   <213> Lactococcus lactis
<400> 1
   gaattcaaaa atataatgct tattttagta ttagtaacca tctctataat tttaaatata 60
   ttaattaacc gatttattat ttttcatcac ttagggataa tgaataatca aattaatatt 120
   gatagtatat taagttcttt atcatgttta ggaaaaattt ttggtattgc cttattagcc 180
   cccatactcg aagaaagtat tttcagagcg tctatttacc aaatcttcat taatgataaa 240
   gtttcttttc ttatctctag cttactattt gcatttttac ataggggtta tagttgggtt 300
   ttcttcacgt atctgccagt aagtttatgt atgacattta tctatcatcg cagaaaaata 360
   ttgacagatt ccattctatt tcattcgtta tttaatttat tagtattggg tttgaatttt 420
   ttaatatgaa ataaatttta gaatagtact tactttttgg ataaatagta aaattataga 480
   aacgattcat tattggttct cagatgtcta tagagttgga cactttcagt gtttagataa 540
   aaattaggac taacaagtaa ctactgaaat attaaccaat tatagtttat aaaaaaacga 600
   aggataaata tacatgctag acattttaaa taaagcaaga atacataaaa aatggttcct 660
   attttcatat tcaattatct ctttttgtat tacaattatt tatattgttt ttaatcacac 720
   attttttaaa gttaattggg caaaatataa tagcgatgac agctataaaa ataaagtaga 780
   tgagatttta aagcatggag ttttctggat taatggaaat ttaacatcta ttagttcgcc 840
   attattaatt tgccttttct tgcttggtgc attcttttca ttaactattt tcttcttaac 900
   ttggagaaat ttatcgacta gaacatggac cccaattata tcctttcttg gatttctgat 960
   tccatttatt catagtgatg gaaatttcat aaatttattg attttatctt ttattcttat 1020
   actatttggg gctatttcct ctgttcctag tcttagatat ttttaaatat tacagcccaa 1080
   aatgaatact taaaaatatc attcactctt tatatgctaa tacccttaag aagtctcaaa 1140
   tacgaacgaa aaatattcta atagggtcat ctatcacata aatctactct atgctaaaaa 1200
   caaaaatctt atttaataat tatattctca tttctatctg tagtgtttat taatattttt 1260
   gaaagataaa gatagaaaga attaatcatt aaactatcag aaattacaaa aatggctagc 1320
   atactgctta gccattttta ttttaattct gcgaaccgag ggggttaagg gtggagcttt 1380
   gctccccctt acaagcgcca caatagccac gaagtggcta gcttgtgggt tgcttgccaa 1440
   gactttatct ttattctagc ttttgagggt cgagcttgag cgtcggacac gaaaagtgct 1500
   agaataaaga tatggacgga acgtccatgg aaaggcgggg gttatgagcg aacacttaaa 1560
   tatggctagc attaaaaaga aacaaccaaa tcgaaaagaa cgaaaacaaa taagtttcag 1620
   agtgagcgaa ccggaatatt taaaccttga gcgctcagcg aaagtcttaa atatttcggt 1680
   gccggctttt gtcaaaaaga aggcacaagg cgctcgtgtc gtcgcaccta aaattaatcc 1740
   agacgattca aaagaaatgg ctcgccagtt ggcagcactt ggcaataacg tgaatcaact 1800
   cgctaaaagg gtcaatcaga ttgaatttgc ggataaggac acgcaagagc gcctatcagc 1860
   cgatttaagg cgcaccttac acggtctggg ggaaatatgg cgacaactca cataaaacgc 1920
   tcaaatggcg cttctagact cgtcaactac gctgaaaaaa gagcggttca aaaagacggc 1980
   tataatttag acattgagta tgccaaatct gaactcaaac aagttcgaga aatttacgga 2040
   aacaaagggg caacgcaagc ctacgcttca agagtggcat tctctccgaa agaatttgac 2100
   cctaaaaatg taaaagacca actaaaggca ctagaaatcg ctaaagaaat ctattcaacc 2160
   gcctatccca accaacaaat cgcaatgtat gttcacaacg acaccgattc cctccacgtt 2220
   cacgccgtga ttggcgccat taacctacta acaggtaaaa aaatgcacgg caattggcaa 2280
   gaataccgtg aaaggctcgt taaaataacg gataaagtcg tggagaaaca tggcttaacc 2340
   gtaaccgttc ctcatccgcg acctgaaaaa agaaccatgg cagaactaaa aatgaaagcc 2400
   cgcggacaag tcacctggaa agacaaaatc agacaagccg tcgatacaac catgcgagaa 2460
   gctcatatta gcgattttaa gagctttaaa gagaaacttg gtgaactagc cgtcaatgtc 2520
   attgaacgtg gcagagacct cacatatact ctcacaggca ctgattataa atcacggggc 2580
   gcaaaactcg gagaggatta caaaaaggag accatttttt atgagctgga cagaagaaac 2640
   caattacagt acggaacaag tcgacaacga caaggtcgcg cttggcttga aggacgtgga 2700
   gaacgccttg aacaagaaca acgcgctcgt caaaaccttg caaaaagagc agaagaccta 2760
   caaagaagaa ctctcgaaag cactgaacaa tcaattcaac caagccatca acgacctcaa 2820
   aaatcaaaag aaagaggact gggagggcct agcctctaat ttcgttaatc gcctcaatga 2880
   cagcactaaa gaaatcacga atagccagct tgaaacggca caagaggaga tagacaagaa 2940
   ctttgcacaa aaagaacaac gcttaaataa ccttgttttt aacattgaga gcgccgaaca 3000
   agccttaaat tttctaaata acagaatcaa tcacatcaaa tcaaccgaaa gattgcagaa 3060
   acaaaagcag ttctttcaag aaatcgtctt tattttgggc gcaatcatgg caagcctcgg 3120
   aacattactc ttggtctttg ccttttgttc tgccctttat ggctttgggt ggcattccat 3180
   ttggaactgg aaacaaatta ctccagtcta tgatgctcca acctttcaaa aaggattatt 3240
   tatcgtcatt aaagccattt taagtcttct attcttcatt tttggggtac tcgttatgtt 3300
   tttccccttg gcgatttacc acaaattcct aaatagaatc aaaaatagtt acagaggttt 3360
   tgggggatgg ctcaataaag tattcatcag aaagtaagac agtttttctg tctttttttt 3420
   attgatcact tcgcaaaatt cgcgagcaca aaaattaaag ataatgcaaa ttaaaaattt 3480
   cgtgttgtga gccttggcga acttttcctt ttggcaacct cggagagtgg gggaattttt 3540
   gcgaaagcaa aaagggggca aagcccctta aaatgctttt gggaaaaatc tatgattttt 3600
   gtccttttta aacctctttt ttcagaaggg gggaaattta aaaaatgagg ctgaaaaatc 3660
   cgagggtctc ttttatattt cttttataaa tcttttaaac ctcttttagg gggctgggaa 3720
   acgttgatat cactagcgtg aagcgttggt tacatacccc taaatagggt actacatacc 3780
   cctaaatagg gtactacata cccctaaata gggtactaca tacccctaaa tagggcgaga 3840
   aagtttataa ccccttttta gggtacttca tttttttata acccttattt agggtacttc 3900
   atttttttat aacccttatt tagggtgaca aaaacccccg ttataaaggt gttttgcttt 3960
   tataacccct ttttagggtg cctctataac ccttatttag ggtagatatt ttatataaaa 4020
   attgctataa tttttataac cctaaaggga taaagaaagg aagtataatg gttcatgaaa 4080
   tagtacaata tcacaacgat tttaacactg ttccacttag aggatttaat gaacgagaac 4140
   gtagaattgt aatggcatta cttcatcaag taaaaaataa agatgtcgaa gtggttcaat 4200
   tagactttga tactttgcgt ggattatctg gttggaatga tactttagct aaatctgaaa 4260
   attccaatgc taaatttaac cggtatcttg aaaacttgtc tgataaaatt atgacattac 4320
   gaggaactct aagaagtgaa gatggtttgc aagtagttaa atttagtctc tttccaacat 4380
   ttattattga tgggaaaaat actatgaccc taaaagttca aattaaccct acttttaaat 4440
   atcttactaa tatctttgat atgttcacag cttttgaatt agatgattat aatcgtatga 4500
   acactagcta tgggcaagaa ctttatagat tattaaaaca gtatcgaaca tctggttttt 4560
   atcgtgtgaa gatagaggac ttgcgacatc tattatcagt tcctgaaagc tataccaatg 4620
   caaaaatgga tcaaaaagta ttttcaaaaa ctactgtaac tgaccttacc aatgcttttc 4680
   cgaattttaa aatcaaacaa gaacgaggca ctggtcgagg tcgaccaata attggttaca 4740
   ccttcacttt cgataaagaa gccccaaata agtatgagct agaccgcaaa aagcaagaac 4800
   aaattgccca attttggaaa tcaaatgacc ctgagccaat gcctaatgca gttgctcaaa 4860
   cggaatatca aaatcctgaa ttacgaaaag aaaaagaaga gctcgaaaaa cataacgcta 4920
   gttttggaga cttattaaag ggctggttca aaaaatagat aaatatgaaa tttaaaaaga 4980
   aaaattatac tcctcaagta gatgaaaaag actgtggttg tgcggcatta tcaatgattt 5040
   taaaaactta cgaaacagaa aagtcacttg cttcattttt attgaatcag aggataaaaa 5100
   tgcataaagt atttgaaaaa attattacaa ttttttttgc ctttttttta tttttcattt 5160
   ctcaaatccc aatatactac gtaaattata aaaataaaga aaataattta tatggaatat 5220
   caaataaaat atcattacct tttatattta ttgctttatt tgttattata atagcagttg 5280
   ctctaggtaa aaaaagagga ttttaccatc attcgaagaa aacattagaa ttc        5333
<210> 2
   <211> 2590
   <212> ADN
   <213> Lactococcus lactis
<400> 2
   cataaatcta ctctatgcta aaaacaaaaa tcttatttaa taattatatt ctcatttcta 60
   tctgtagtgt ttattaatat ttttgaaaga taaagataga aagaattaat cattaaacta 120
   tcagaaatta caaaaatggc tagcatactg cttagccatt tttattttaa ttctgcgaac 180
   cgagggggtt aagggtggag ctttgctccc ccttacaagc gccacaatag ccacgaagtg 240
   gctagcttgt gggttgcttg ccaagacttt atctttattc tagcttttga gggtcgagct 300
   tgagcgtcgg acacgaaaag tgctagaata aagatatgga cggaacgtcc atggaaaggc 360
   gggggttatg agcgaacact taaatatggc tagcattaaa aagaaacaac caaatcgaaa 420
   agaacgaaaa caaataagtt tcagagtgag cgaaccggaa tatttaaacc ttgagcgctc 480
   agcgaaagtc ttaaatattt cggtgccggc ttttgtcaaa aagaaggcac aaggcgctcg 540
   tgtcgtcgca cctaaaatta atccagacga ttcaaaagaa atggctcgcc agttggcagc 600
   acttggcaat aacgtgaatc aactcgctaa aagggtcaat cagattgaat ttgcggataa 660
   ggacacgcaa gagcgcctat cagccgattt aaggcgcacc ttacacggtc tgggggaaat 720
   atggcgacaa ctcacataaa acgctcaaat ggcgcttcta gactcgtcaa ctacgctgaa 780
   aaaagagcgg ttcaaaaaga cggctataat ttagacattg agtatgccaa atctgaactc 840
   aaacaagttc gagaaattta cggaaacaaa ggggcaacgc aagcctacgc ttcaagagtg 900
   gcattctctc cgaaagaatt tgaccctaaa aatgtaaaag accaactaaa ggcactagaa 960
   atcgctaaag aaatctattc aaccgcctat cccaaccaac aaatcgcaat gtatgttcac 1020
   aacgacaccg attccctcca cgttcacgcc gtgattggcg ccattaacct actaacaggt 1080
   aaaaaaatgc acggcaattg gcaagaatac cgtgaaaggc tcgttaaaat aacggataaa 1140
   gtcgtggaga aacatggctt aaccgtaacc gttcctcatc cgcgacctga aaaaagaacc 1200
   atggcagaac taaaaatgaa agcccgcgga caagtcacct ggaaagacaa aatcagacaa 1260
   gccgtcgata caaccatgcg agaagctcat attagcgatt ttaagagctt taaagagaaa 1320
   cttggtgaac tagccgtcaa tgtcattgaa cgtggcagag acctcacata tactctcaca 1380
   ggcactgatt ataaatcacg gggcgcaaaa ctcggagagg attacaaaaa ggagaccatt 1440
   ttttatgagc tggacagaag aaaccaatta cagtacggaa caagtcgaca acgacaaggt 1500
   cgcgcttggc ttgaaggacg tggagaacgc cttgaacaag aacaacgcgc tcgtcaaaac 1560
   cttgcaaaaa gagcagaaga cctacaaaga agaactctcg aaagcactga acaatcaatt 1620
   caaccaagcc atcaacgacc tcaaaaatca aaagaaagag gactgggagg gcctagcctc 1680
   taatttcgtt aatcgcctca atgacagcac taaagaaatc acgaatagcc agcttgaaac 1740
   ggcacaagag gagatagaca agaactttgc acaaaaagaa caacgcttaa ataaccttgt 1800
   ttttaacatt gagagcgccg aacaagcctt aaattttcta aataacagaa tcaatcacat 1860
   caaatcaacc gaaagattgc agaaacaaaa gcagttcttt caagaaatcg tctttatttt 1920
   gggcgcaatc atggcaagcc tcggaacatt actcttggtc tttgcctttt gttctgccct 1980
   ttatggcttt gggtggcatt ccatttggaa ctggaaacaa attactccag tctatgatgc 2040
   tccaaccttt caaaaaggat tatttatcgt cattaaagcc attttaagtc ttctattctt 2100
   catttttggg gtactcgtta tgtttttccc cttggcgatt taccacaaat tcctaaatag 2160
   aatcaaaaat agttacagag gttttggggg atggctcaat aaagtattca tcagaaagta 2220
   agacagtttt tctgtctttt ttttattgat cacttcgcaa aattcgcgag cacaaaaatt 2280
   aaagataatg caaattaaaa atttcgtgtt gtgagccttg gcgaactttt ccttttggca 2340
   acctcggaga gtgggggaat ttttgcgaaa gcaaaaaggg ggcaaagccc cttaaaatgc 2400
   ttttgggaaa aatctatgat ttttgtcctt tttaaacctc ttttttcaga aggggggaaa 2460
   tttaaaaaat gaggctgaaa aatccgaggg tctcttttat atttctttta taaatctttt 2520
   aaacctcttt tagggggctg ggaaacgttg atatcactag cgtgaagcgt tggttacata 2580
   cccctaaata                                                        2590
<210> 3
   <211> 31
   <212> ADN
   <213> Séquence artificielle
<220>
   <223> Description de la séquence artificielle:oligonucleotide
<400> 3
   tacatacgcg tctcatatat actttagatt g                                31
<210> 4
   <211> 31
   <212> ADN
   <213> Séquence artificielle
<220>
   <223> Description de la séquence artificielle:oligonucleotide
<400> 4
   tacatacgcg tgacttagaa gcaaacttaa g 31
<210> 5
   <211> 31
   <212> ADN
   <213> Séquence artificielle
<220>
   <223> Description de la séquence artificielle:oligonucleotide
<400> 5
   ttaaatgatc agagctccac cgcggtggcg g 31
<210> 6
   <211> 31
   <212> ADN
   <213> Séquence artificielle
<220>
   <223> Description de la séquence artificielle:oligonucleotide
<400> 6
   tattttgatc agaacaaaag ctgggtaccg g 31
<210> 7
   <211> 31
   <212> ADN
   <213> Séquence artificielle
<220>
   <223> Description de la séquence artificielle:oligonucleotide
<400> 7
   atttatgatc atttccagtc gggaaacctg t 31
<210> 8
   <211> 31
   <212> ADN
   <213> Séquence artificielle
<220>
   <223> Description de la séquence artificielle:oligonucleotide
<400> 8
   aattttgatc aagtatacct aataatttat c 31
<210> 9
   <211> 31
   <212> ADN
   <213> Séquence artificielle
<220>
   <223> Description de la séquence artificielle:oligonucleotide
<400> 9
   tatgtgaatt cgacttagaa gcaaacttaa g 31
<210> 10
   <211> 31
   <212> ADN
   <213> Séquence artificielle
<220>
   <223> Description de la séquence artificielle:oligonucleotide
<400> 10
   tatgtgaatt cagtatacct aataatttat c 31
<210> 11
   <211> 29
   <212> ADN
   <213> Séquence artificielle
<220>
   <223> Description de la séquence artificielle:oligonucleotide
<400> 11
   tataagaatt ccgctcgtgt cgtcgcacc 29
<210> 12
   <211> 29
   <212> ADN
   <213> Séquence artificielle
<220>
   <223> Description de la séquence artificielle:oligonucleotide
<400> 12
   tataagaatt ccataaatct actctatgc 29
<210> 13
   <211> 30
   <212> ADN
   <213> Séquence artificielle
<220>
   <223> Description de la séquence artificielle:oligonucleotide
<400> 13
   tataagaatt cgtagatgag attttaaagc 30
<210> 14
   <211> 29
   <212> ADN
   <213> Séquence artificielle
<220>
   <223> Description de la séquence artificielle:oligonucleotide
<400> 14
   tattagaatt ctatttaggg gtatgtaac 29
<210> 15
   <211> 32
   <212> ADN
   <213> Séquence artificielle
<220>
   <223> Description de la séquence artificielle:oligonucleotide
<400> 15
   tattagaatt ccgtgatttc tttagtgctg tc 32
<210> 16
   <211> 31
   <212> ADN
   <213> Séquence artificielle
<220>
   <223> Description de la séquence artificielle:oligonucleotide
<400> 16
   tatataagct tagtatacct aataatttat c 31
<210> 17
   <211> 29
   <212> ADN
   <213> Séquence artificielle
<220>
   <223> Description de la séquence artificielle:oligonucleotide
<400> 17
   tatataagct tcacttcgca aaattcgcg 29
<210> 18
   <211> 29
   <212> ADN
   <213> Séquence artificielle
<220>
   <223> Description de la séquence artificielle:oligonucleotide
<400> 18
   tatataagct tgagggtcga gcttgagcg 29
<210> 19
   <211> 31
   <212> ADN
   <213> Séquence artificielle
<220>
   <223> Description de la séquence artificielle:oligonucleotide
<400> 19
   tatataagct tcactcttta tatgctaata c 31
<210> 20
   <211> 20
   <212> ADN
   <213> Séquence artificielle
<220>
   <223> Description de la séquence artificielle:oligonucleotide
<400> 20
   aggtttcccg actggaaatg 20
<210> 21
   <211> 31
   <212> ADN
   <213> Séquence artificielle
<220>
   <223> Description de la séquence artificielle:oligonucleotide
<400> 21
   tacgtgaatt cagttttaaa tcaatctaaa g 31
<210> 22
   <211> 31
   <212> ADN
   <213> Séquence artificielle
<220>
   <223> Description de la séquence artificielle:oligonucleotide
<400> 22
   tacgtggatc catcggcata atcgttaaaa c                                    31
<210> 23
   <211> 31
   <212> ADN
   <213> Séquence artificielle
<220>
   <223> Description de la séquence artificielle:oligonucleotide
<400> 23
   tacgtgaatt cagaagaacc cttaactaaa c                                    31